(19) Europäisches Patentamt
European Patent Office
Office européen des brevets

(11) Publication number: 0 328 498 A2

# (12) EUROPEAN PATENT APPLICATION

(21) Application number: 89850020.2

(22) Date of filing: 24.01.89

(51) Int. Cl.$^4$: C 07 C 59/125
C 07 C 59/13, C 07 C 69/708,
C 07 C 103/38, C 07 C 93/00,
C 11 D 1/02

(30) Priority: 08.02.88 SE 8800395

(43) Date of publication of application:
16.08.89 Bulletin 89/33

(84) Designated Contracting States: DE FR GB IT

(71) Applicant: SKF Nova AB
S-415 50 Göteborg (SE)

(72) Inventor: Lindsten, Göran
Torsgatan 25
S-431 38 Mölndal (SE)

Wyman, John
17 Monadnock Drive
Westford Massachusets 01886 (US)

## (54) Anionic compounds derived from non-ionic surface active agents and compositions containing anionic compounds derived from non-ionic surface active agents.

(57) Chemical compounds of the formula:
A - X - B
wherein A is derived from a non-ionic surface active agent and is dissolved by a high molecular weight non-polar oil or a polar lubricant when said compound is bonded to the metal, B is an organic carboxylic acid group and X is a connecting group linking A to B wherein X comprises at least two carbon atoms.

EP 0 328 498 A2

**Description**

# ANIONIC COMPOUNDS DERIVED FROM NON-IONINC SURFACE ACTIVE AGENTS AND COMPOSITIONS CONTAINING ANIONIC COMPOUNDS DERIVED FROM NON-IONINC SURFACE ACTIVE AGENTS

## BACKGROUND OF THE INVENTION

The present invention relates to novel compounds having one end which bonds strongly to metal surfaces and a "tail" which is dissolved by and which is compatible with hydrocarbon or synthetic ester oils. The tail of the compound may be designed to be soluble either in polar oils or in high molecular weight hydrocarbon non-polar oils.

Retention of oils on metal surfaces, such as lubricating oils and oils to prevent metal corrosion may be improved by use of compounds of the present invention as additives in the oil. By retaining oil at the metal surface, lubrication and corrosion inhibition provided by the oil may be improved. Compounds of the present invention may also be used as dispersants for magnetic particles in magnetic fluids known as superparamagnetic liquids.

The addition of certain acids, particularly fatty acids such as oleic acid, at a low molecular weight non-polar hydrocarbon oil such as kerosene or xylene, in some instances, help retain the oil on the metal surface. It is believed that carboxyl groups bond strongly to metal surfaces and the "tail" of the acid extends up and away from the metal surface. When the tail of the acid is dissolved by the oil, oil molecules remain associated with the tail near the metal surface. The association of oil molecules with the acid tails retains the oil molecules at the metal surface, thereby improving the lubricating and corrosion inhibiting effects of the oil.

To improve the retention of oil at a metal surface the tail of the acid must be carefully designed to be compatible with and dissolved by the particular oil when the acid is attached to the metal surface. The "tail" of oleic acid, for instance, is dissolved by many low molecular weight non-polar hydrocarbon oils and solvents, such as xylene and kerosene, but not by certain high molecular weight oils such as 6 or 8 centistoke (cst) poly(alpha olefin) oils commonly used as synthetic based hydrocarbon lubricating oils.

Fatty acids, such as oleic acid, have also been used to suspend magnetic particles in low molecular weight non-polar carrier liquids such as kerosene or xylene. Magnetite ($Fe_3O_4$), for instance, can be coated by oleic acid in alkaline aqueous suspension, to render the magnetite particle hydrophobic. When the coated magnetite is treated with a low molecular weight non-polar hydrocarbon, such as heptane, octane, toluene or xylene, the oleic acid "tails" are dissolved by the liquid and the coated magnetite spontaneously peptizes to form a stable colloidal suspension. This demonstrates that the tail of oleic acid, when it is attached to the magnetite, is soluble in these oils. However, magnetite coated with oleic acid will not peptize in a poly(alpha olefin) oil and will not form a stable colloid in this liquid. This shows that the oleic acid tail is not well matched to a poly(alpha olefin) hydrocarbon oil when the acid is bonded to magnetite. This indicates that oleic acid may not materially improve retention of poly(alpha olefin) oils onto metal surfaces.

Similarly, the oleic acid "tail" is not dissolved by a polar liquid ester such as dioctyl sebacate. This is demonstrated by the fact that magnetite particles coated with oleic acid will not form a stable colloidal suspension in dioctyl sebacate. Accordingly, $C_{18}$ carboxylic acids, such as oleic acid, are not satisfactory additives for polar liquid ester lubricants.

Additives have often been included in high molecular weight oils to help retain oil molecules at a metal surface, but many of these additives have either not been sufficiently soluble in the oil or have not bonded adequately to the metal surface. It is a purpose of the present invention to provide chemical compounds specifically designed 1) to bond strongly to the metal surface and 2) to be dissolved by the oil when the material is bonded to the metal surface thereby serving to retain the oil at the metal surface. The compounds of the present invention may also be used as dispersants for magnetic particles in high molecular weight non-polar carrier liquids and polar carrier liquids.

## BRIEF DESCRIPTION OF THE DRAWING

Figure 1 is a graph comparing the pH values for Dextrol OC-70, on acid phosphoric acid ester dispersant, with a dispersant of the present invention, dispersant No. 2 from Table 1, as the two dispersants are titrated with sodium hydroxide. The pKa values are calculated from this graph.

## SUMMARY OF THE INVENTION

One embodiment of the present invention are chemical compounds of the formula:

A-X-B

wherein A is derived from a non-ionic surface active agent and is dissolved by a high molecular weight

non-polar oil or a polar lubricant when said compound is bonded to the metal, B is an organic carboxylic acid group and X is a connecting group linking A to B wherein X comprises at least two carbon atoms.

Additional advantages and embodiments of the invention will be set forth in part in the description which follows and in part will be apparent from the description, or may be learned by practice of the invention. The advantages of the invention may be realized and attained by processes, materials and combinations particularly pointed out in the appended claims.

## DETAILED DESCRIPTION OF THE INVENTION

The present invention provides compounds with a carboxyl group which will bond strongly to metal surfaces and with "tails" that are compatible with and dissolved by the oils which are to be used, when the compound is bonded to a metal surface. The general structure of these compounds is shown below:

A-X-B

wherein A is the oil soluble group, B is the carboxyl group, and X is a connector group between A and B having at least two carbon atoms.

Compounds of the present invention may be used as additives in lubricating oils or corrosion inhibiting oils which help retain the oil at the metal surface thereby enhancing the lubricating or corrosion resistant effects of the oils. In addition, compounds of the present invention may be used as dispersants for magnetic particles in superparamagnetic liquids. These uses of the present invention are described in the ensuing paragraphs.

Compounds of the present invention must not only be soluble in the oil, but the tail portion (A group) must be soluble in the oil when the compound is bonded to a metal surface. The solubility characteristics of the A group are apparently altered when the compound is bonded to a metal surface so that a compound that is soluble in an oil when the compound is not bound to metal may be insufficiently soluble in that same oil when the compound is bonded to metal. Oleic acid, for instance, is ordinarily soluble in 6 or 8 centistoke (cst) poly(alpha olefin) oil but when oleic acid is attached to a metal oxide surface, such as magnetite, the tails of oleic acid are not dissolved by the 6 or 8 cst oils. This is demonstrated by the fact that sub-micron sized magnetite does not form a stable suspension in 6 or 8 cst oil when coated with oleic acid. In contrast, oleic acid coated magnetite does form a stable colloidal suspension in light weight oils such as xylene or kerosene indicating that the attached oleic acid is soluble in xylene or kerosene. It is believed that the change in solubility characteristics results, primarily, from the restriction on molecular movement imposed by bonding to the metal which orients the molecule in a fixed location in space.

A similar alteration in solubility of the A group is believed to occur when oleic acid attaches to the metal surface of lubricated machinery. The decrease in solubility of oleic acid in the oil contributes to the inability of oleic acid to effectively retain high molecular weight lubricating oils at the metal surface. Compounds of the present invention may also be used in corrosion inhibiting oils. Synthetic lubricating oils with which compounds of the present invention may be used include dioctyl azelate and "Priolube 3970", a commercially available oil produced by Unichema Chemie of Holland. Other lubricating and corrosion inhibiting oils, of course, may be used in conjuction with compounds of the present invention.

To use compounds of the present invention as oil additives, one may simply stir the additive into the oil at room temperature. The amount of additive placed in the oil is a matter which can readily be determined by one of ordinary skill in the art taking into consideration the purpose intended for the oil and additive combination. Use of compounds of the present invention as dispersants for magnetic colloids is described in detail subsequently in this specification.

The oil soluble group A of the present invention is derived from a non-ionic surface active agent and is selected to be compatible with and dissolved by a specific carrier oil. Non-ionic surface active agents from which A is derived include ethoxylated alcohols, ethoxylated alkyl phenols, ethoxylated fatty acids, ethoxylated amides, ethoxylated amines and ethylene oxide/propylene oxide block polymers. Examples of commercially available non-ionic precursors to the oil soluble A group include, but are not limited to, poly(ethoxylated) alcohols such as "DeSonic 6T" (produced by DeSoto Inc.), poly(ethoxylated) fatty acids such as "Mulgofen VN-430" (produced by GAF Corp.), ethoxylated and poly(ethoxylated) amides such as "Ethomid O/15" (produced by Akzo Chemie BV), ethoxylated and poly(ethoxylated) alkylated phenols such as "Antarox CA-210" and "DM-430" (produced by GAF Corp.). Alcohols reacted with a mixture of propylene oxide and ethylene oxide such as "Tergitol Min-Foam 1X" and "Tergitol Min-Foam 2X" (produced by Union Carbide Corp.) are also precursors to the oil soluble group A of the dispersants in the practice of the present invention.

Specific examples of non-ionic surface active materials useful as precursors of the A group in the present invention are set forth in more detail below. The following structures illustrate non-ionic surface active materials useful in the present invention and are not exhaustive of the non-ionic surface active agents which may be found to be useful:

1) Ethoxylated Alcohols (precursors of compounds preferred as additives for polar lubricating liquids or as dispersants in magnetic colloids using a polar carrier liquid):

$R-O\!-\!(CH_2CHYO)\!-\quad H$

R = saturated or unsaturated hydrocarbon having one to about 25 carbon atoms. R may be a linear or branched, normal, secondary, tertiary, or iso structure but preferably R is a linear or branched alkyl or alkylene

chain with 2-25 carbons. More preferably, R is an alkyl chain with 4-15 carbons, n = 2-10 and Y is hydrogen; Y is hydrogen or methyl; and
n = 1 to about 30.

2) Ethoxylated alkyl phenols:

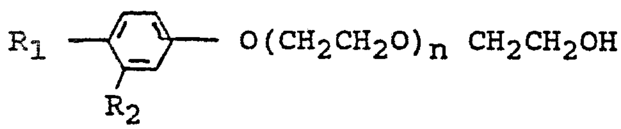

Usually, $R_1$ = tertiary $C_8$, or $C_9$.
$R_2$ = H or $C_8$ or $C_9$; and
n = 1 to about 19.
In most preferred materials $R_2$ = H.

3) Ethoxylated Fatty Acids:

$R - \overset{O}{\overset{\|}{C}} - O + CH_2CH_2O +_n \ CH_2CH_2OH$;
R = $C_{11}$ to about $C_{17}$ representing the alkyl group of lauric, myristic, palmitic, oleic, stearic, or isostearic acid; and
n = 1 to about 19.

4) Ethoxylated Amides

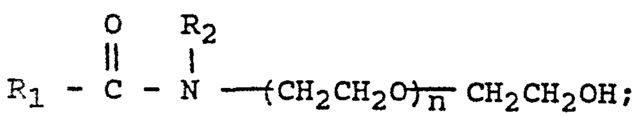

$R_1 - \overset{O}{\overset{\|}{C}}$ - is preferably derived from a fatty acid such as lauric, myristic, palmitic, oleic, stearic, or isostearic acid;
n = 0 to about 29;
$R_2$ = $CH_3$ or $+CH_2CH_2O+_n$ $CH_2CH_2OH$ and is preferably $CH_3$.

5) Ethoxylated Amines

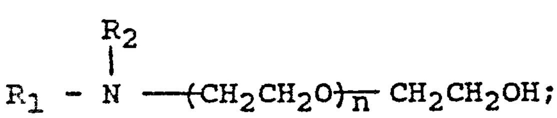

$R_1$ can be an alkyl group with from about 4 to about 25 carbon atoms;
$R_2$ can = $R_1$ or $R_2$ can be $-CH_3$ or $+CH_2CH_2O+_n$ $-CH_2CH_2OH$;
n = 1 to about 29.

6) Ethylene oxide/propylene oxide block polymers (Propylene oxide is the oil soluble group)

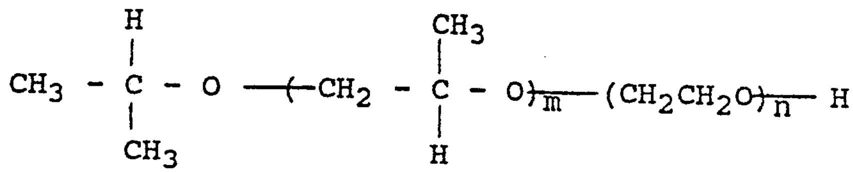

(Propylene oxide) (Ethylene oxide)

m and n are greater than 1 and may be the same or different.

Of course, when the foregoing precursors of A are part of an A-X-B compound of the present invention, their structure will be the same as that identified above except that the H of the terminal OH portion of the precursor will not be present and the X group will be linked to the oxygen of the terminal OH portion of the A group precursor. For instance, when A is derived from ethoxylated alcohols, the formula for A will be $R\text{-}O\text{-}(CH_2CHYO)_n\text{-}$.

Precursors to the oil soluble group A are organic compounds containing hydroxyl groups, preferably alcohols, reacted with ethylene oxide or a mixture of ethylene oxide and propylene oxide. It is known, for example, that an alcohol reacted with six moles of ethylene oxide per mole of alcohol will generate a mixture where there are products in which the alcohol will have combined with from about three to about nine ethylene oxide units. The major portion of the mixture consists of alcohol which as reacted with six ethylene oxide units.

Non-ionic surface active agents which are commercially available and may be useful as a precursor of A are described in McCutcheons Annual, 1987, Emulsifiers and Detergents", North American and International Edition, MC Publishing Company, Glen Rock, New Jersey, U.S.A., pages 118-140, the disclosure of which is incorporated herein by reference. These materials generally form compounds having an A group which is dissolved by polar liquid ester oils. The most preferred materials for use with polar liquid ester lubricants are ethoxylated alcohols identified above.

To achieve solubility in and compatibility with high molecular weight non-polar hydrocarbon oils, the A group is preferably derived from a linear or branched alcohol with from 2 to 25 carbon atoms, a fatty alcohol such as oleyl alcohol or isostearyl alcohol or other high molecular weight alcohol with up to 25 carbon atoms, or an alkylated aromatic compound.

When mixtures resulting from the reaction of alcohol and ethylene and/or propylene oxide are reacted with the X group precursor, A-X-B compounds with different molecular lengths are formed. This irregularity in molecular length may be advantageous for certain applications, e.g., when using the A-X-B compounds as dispersants in superparamagnetic liquids.

The structure of the X group which connects the oil soluble group with the carboxyl group may be selected for convenience in compound synthesis or to enhance the physical or chemical properties of the compound. In general, for convenience in compound synthesis, the precursor of the connecting group is selected so that by chemical reaction of the A group precursor with the X groups precursor, the compound with the general structure A-X-B is formed directly. Structures of X which may be useful in the present invention are illustrated by the following formulae:

$$-\overset{O}{\overset{\|}{C}}-(CH_2)_p- \qquad p= 1 - 8;$$

or $$-(CH_2)_q- \qquad q= 2 - 8;$$

or

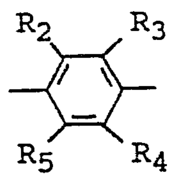

wherein $R_2$, $R_3$, $R_4$ and $R_5$ can be the same or different and may be hydrogen, alkyl groups with 1 to 25 carbons, halogen or additional A groups with A being any of the A group substituents described in the foregoing paragraphs.

The direct formation of an A-X-B compound of the present invention is illustrated by the reaction of "DeSonic 6T", a mixture of poly(ethoxylated) alcohols available from DeSoto Inc. Reaction of "DeSonic 6T", which is a mixture of compounds produced by reacting tridecyl alcohol with six moles of ethylene oxide, with a stoichiometric amount of succinic anhydride, produced directly a compound of the present invention with the general structure given below:

$RO-(CH_2CH_2O)_n\overset{O}{\overset{\|}{C}}CH_2CH_2\overset{O}{\overset{\|}{C}}OH.$

The X group in the above formula is

$-\overset{O}{\overset{\|}{C}}CH_2CH_2-$, and the B group is COOH. When the A group derives from "DeSonic 6T", R is a linear $C_{13}$ alkyl group and n has an average value of six. This structure is particularly useful in connection with magnetic colloids with a polar carrier liquid such as dioctyl phthalate or polar lubricants such as dibutyl sebacate.

Other chemicals, particularly glutaric anhydride, can be used in place of succinic anhydride.

To provide enhanced oxidative stability of A-X-B compound used as oil additives or as a dispersant in a superparamagnetic colloid, the X group can be aromatic or substituted aromatic substituent. In an embodiment with an aromatic X group, up to five A groups can be included in the compound, the structure of which is illustrated below:

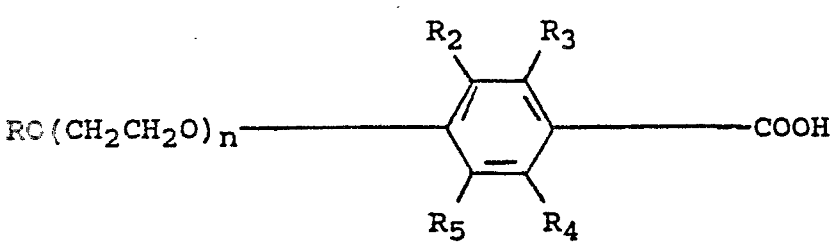

where the A group is $RO-(CH_2CH_2O)-$, the X group is the aromatic group, and the B group is COOH. R may be a linear or branched alkyl or alkylene chain with 2-25 carbons or an alkylated aromatic group and n is at least 1. $R_2$, $R_3$, $R_4$ and $R_5$, which may be the same or different, are hydrogen, alkyl groups with 1-25 carbons, halogen or additional $RO-(CH_2CH_2O)-$ groups. Compounds of the preceding formula can be prepared by reacting the corresponding chlorinated alcohol with a benzoic acid derivative, preferably 4-hydroxy benzoic acid.

A-X-B compounds wherein X is aromatic may also be illustrated by the following formula:

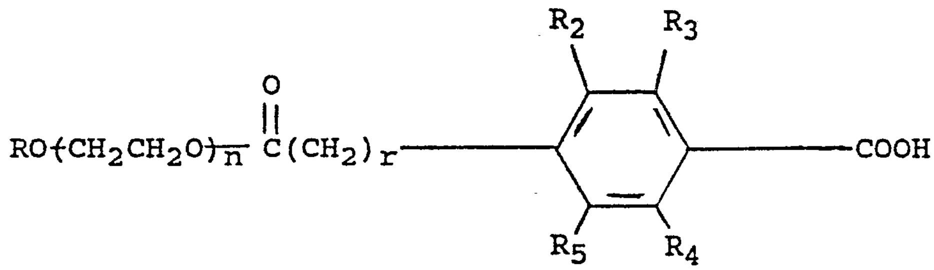

where the A group is $RO\text{-}(CH_2CH_2O)_n\text{-}$ ;
the X group is:

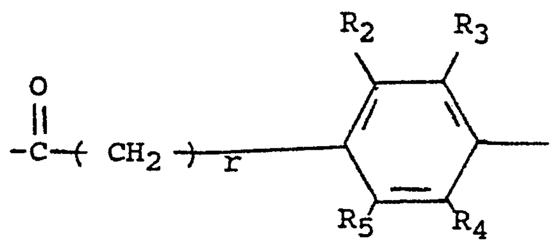

r may be 0 to about 8 and is preferably 0 or 1, and the B group is COOH. R again may be linear or branched alkyl or alkylene chain with 2-25 carbons or an alkylated aromatic group and n is at least 1. As explained above, $R_2$, $R_3$, $R_4$, and $R_5$ may be the same or different and may be hydrogen, alkyl groups with 1-25 carbons, halogen or additional A groups.

Commercially available ether carboxylic acids, such as those produced by Chemische Fabrik CHEM-Y GmbH under the general name "Akypo" are also useful dispersants for the practice of our invention and may also form useful oil additives. The general formula of "Akypo" is believed to be illustrated by the following formula:

$$R_1O\text{-}(CH_2CH_2O)_n\text{-}\ CH_2\ \overset{O}{\overset{\|}{C}}OH$$

where the A group is $R_1O\text{-}(CH_2CH_2O)_n\text{-}$ , the X group is $CH_2$, and the B group is COOH. $R_1$ is believed to be an alkyl group. Although "Akypo", in which X is a single carbon atom, is a known material, it has been used as a wetting agent and has not previously been used as an oil additive or as a dispersant for a magnetic colloid. Accordingly, use of "Akypo" as a dispersant or as an oil additive is believed to be novel and nonobvious. Oils with which Akypo may be combined include dioctyl azelate and "Priolube 3970".

Other ether carboxylic acids in which the $-(CH_2)_n-$group, corresponding to the X group of the compounds of the present invention, contains up to 8 or more carbon atoms, can be readily prepared by synthetic procedures well known to those skilled in the art.

In general, an alcohol reacted with six moles of ethylene oxide per mole of alcohol will be a mixture in which the alcohol will have combined with from about three to about nine ethylene oxide units. The major portion of the mixture consists of alcohol which has reacted with six ethylene oxide units. When these mixtures are reacted with an X group precursor, A-X-B compounds with different molecular lengths are formed. These materials, attached to metals or magnetic particles, will produce an irregularity in the coating which will inhibit association of the A groups with one another, a phenomenon sometimes referred to as "crystallization".

The structure of the X group may be selected to achieve certain desirable properties, such as oxidation resistance. The X group may also be a perfluorinated chain, preferably a perfluorinated chain having 2-12 carbo atoms, or an aromatic ring where the remaining hydrogen atoms have been replaced by fluorine.

A reaction used to form compounds of the present invention occurs according to the following general formula:

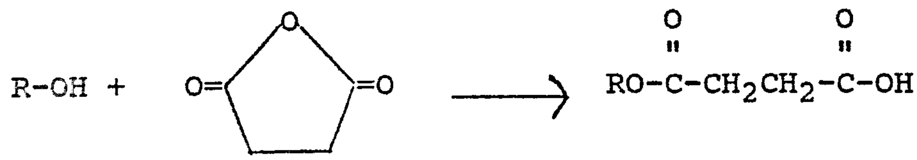

In the foregoing formula, of course, R-OH represents the A group precursor and succinic anhydride contributes the X and B groups of the compound. The product obtained is a succinic acid half ester. A method for preparing compounds of the present invention in which succinic anhydride is used as the X-B group precursor is described in more detail in Example 1. Glutaric anhydride may be used instead of succinic anhydride.

Furthermore, reaction of "DeSonic DA-4", a mixture of compounds produced by reacting one mole of decyl alcohol with four moles of ethylene oxide with the stochimetric amount of succinic anhydride, produced a compound with the general structure:

$$RO\text{-}(CH_2CH_2O)_n\text{-}\overset{O}{\overset{\|}{C}}\,CH_2CH_2\text{-}\overset{O}{\overset{\|}{C}}\,OOH$$

wherein R is a linear $C_{10}$ alkyl group and n has an average value of four.

When compounds of the present invention are used as dispersants for magnetic particles in a carrier liquid, they attach to a particle surface, such as magnetite, and when the A portion of the molecule is dissolved by the carrier liquid, provide a magnetic colloid. Other particles which may be used to form magnetic colloids include zinc ferrite, manganese ferrite, metals such as iron, nickel or cobalt, or chromium dioxide. The ensuing paragraphs and examples explain how compounds of the present invention may be used as dispersants in magnetic colloids.

Carrier liquids useful in the practice of our invention are those liquids which do not form a superparamagnetic liquid with oleic acid coated magnetic particles. This requirement eliminates non-polar low molecular weight oils such as kerosene or xylene. The carrier liquid may be a polar or a high molecular weight non-polar liquid. Non-polar liquid hydrocarbons which may be useful as carrier liquids in the practice of our invention include, but are not limited to, synthetic or natural lubricating oil base stocks such as the alpha olefin oligomers and the 100-, 150-, 500-, and 600- neutral base oils. These materials are believed to be available commercially from Mobil Oil Company. Polar organic liquids useful in the present invention include esters, ketones, ethers, alcohols and water.

The carrier liquid must also be a thermodynamically good solvent for A. The solvent characteristics of particular carrier liquids will be determined largely by experience. Whether or not a particular carrier liquid will be a thermodynamically good solvent for A may also be predicted in accordance with principles discussed in "Dispersion Polymerization in Organic Media", K.E.J. Barrett, Editor, John Wiley & Sons, printed in Great Britain by J.W. Arrowsmith, Ltd. (1975) pages 50-51, the disclosure of which is incorporated herein by reference.

When the carrier liquid is non-polar liquid hydrocarbon oil, the oil soluble group A is preferably a residue from a linear or branched, saturated or unsaturated, alcohol with from 2 to 25 carbon atoms, a fatty alcohol such as oleyl alcohol, or an alkylated aromatic compound.

Polar carrier liquids useful in the present invention are preferably polar esters which include, but are not limited to, those formed from monobasic organic acids and monohydric alcohols. Organic acids which may be used include monobasic acids such as acetic, benzoic, caproic, caprylic, capric, lauric, myristic, palmitic, oleic, stearic, and isostearic acids, dibasic organic acids such as adipic, azeleic, dimer, suberic, succinic, ortho-, meta-, and terephthalic acids, tribasic acids such as citric, trimer, and trimellitic acids, and tetrabasic acids as pyromellitic acid. The alcohols that may be used to prepare these esters include, but are not limited to, monohydric alcohols with from one to about 25 carbon atoms and include normal, secondary, tertiary, and isostructures, they may be saturated or unsaturated, linear or branched, and may be ethoxylated and/or propoxylated. They may include alcohols produced as a result of the oxo- or Ziegler-process. The esters may be prepared from a single alcohol or a mixture of two or more alcohols.

Preferred polar liquids are trimethylolpropane mixed alkanoic acid triesters, mixed alkyl trimellitate triester, dialkyl sebacate and alkyl oleate. Trimethylolpropane mixed alkanoic acid triester is the most preferred carrier liquid, particularly with dispersants derived from ethoxylated alcohols.

Esters useful in the present invention may also be prepared from polyhydric alcohols and monobasic organic acids. Polyhydric alcohols which can be used include but are not limited to ethylene glycol, propylene glycol, 1,3-propanediol, butylene glycol, 1,4-butanediol, glycerine, diethylene glycol, triethylene glycol, dipropylene glycol, tripropylene glycol, pentaerythritol, and trimethylolpropane. The esters may be prepared from a single monobasic organic acid or from a mixture of two or more monobasic acids.

Ketones which are useful as carrier liquids in the practice of our invention include but are not limited to acetone, methyl ethyl ketone, methyl isobutyl ketone, cyclopentanone, and cyclohexanone.

Ethers which are useful as carrier liquids in the practice of our invention include but are not limited to simple

ethers such as diethyl ether, diethylene glycol dimethyl ether, diethylene glycol dibutyl ether, and cyclic ethers such as tetrahydrofuran and dioxane.

Alcohols which are themselves useful as carrier liquids in the practice of our invention include, but are not limited to, those listed above as useful for the preparation of esters used as carrier liquids in the practice of the present invention.

A simple test can be used to determine if a carrier liquid is useful in the practice of our invention. A quantity of about 50 ml of a superparamagnetic liquid with a saturation magnetization of about 200 gauss consisting of fatty acid coated magnetite, preferably oleic acid coated magnetite, in hexane (see Procedure for Preparing Superparamagnetic Liquid Consisting of Fatty Acid Coated Magnetite) is mixed with about 50 ml of the liquid to be used as a carrier liquid in the practice of our invention and placed in a 250 ml beaker. The mixture is stirred and heated in a stream of air to evaporate the hexane and the beaker is placed over a samarium-cobalt magnet (cylindrical, diameter 25 mm and height 10 mm) and placed in a 65°C oven for 24 hours. After cooling, the liquid is poured off from the residue on the bottom of the beaker while the magnet is held in place under the beaker. If substantially all of the coated magnetite remains at the bottom of the beaker, the carrier liquid is likely to be useful in the practice of our invention since it did not form a stable superparamagnetic liquid with the fatty acid coated magnetite.

In practicing the present invention, magnetic particles may be stably suspended in the carrier liquid when they are coated only with dispersants of the present invention. It is often preferable, however, to coat the magnetic particles with a $C_{18}$ monocarboxylic acid, such as oleic, isostearic, linoleic or linolenic acids, preferably oleic acid, peptize the fatty acid coated particles into a low molecular weight hydrocarbon, and subsequently coat the particles with dispersants of the present invention. This preliminary coating with oleic acid followed by allowing the coated magnetite to peptize into a low molecular weight hydrocarbon, rapidly and conveniently separates the magnetite from water and by-product ammonium salts which otherwise must be eliminated by tedious multiple washings with water. The preliminary coating with a $C_{18}$ carboxylic acid may be accomplished in accordance with the following procedure.

## PROCEDURE FOR PREPARING SUPERPARAMAGNETIC LIQUID CONSISTING OF MAGNETITE COATED WITH A $C_{18}$ MONOCARBOXYLIC ACID

In a 1000 ml beaker was placed ferric chloride hexahydrate (1.93 mol, 521.7 g, Merck) and water to make about 600 ml. This mixture was heated until all solids were dissolved. To the resultant solution was added ferrous sulphate heptahydrate (1.0 mol, 278 g) and water to make about 900 ml and this mixture was stirred until all solids were dissolved. This solution was allowed to cool to about 25°C during which time a 3 l beaker equipped with a mechanical stirrer was prepared with 25%-wt ammonium hydroxide solution (750 ml) and water (250 ml). To this stirred ammonium hydroxide solution was added the above prepared iron salt solution during which addition the temperature of the mixture rose to about 60°C as a result of released heat of crystallization of the magnetite. Stirring was continued for about 20 minutes and then oleic acid (0.16 mol, 44.6 g) was added to the magnetite slurry and stirring was continued for another 20 minutes. To this slurry was added a low molecular weight hydrocarbon (150 ml, Shellsol T produced by Shell Oil Co.) and the mixture was stirred well and then allowed to separate. The resulting black colored organic phase was siphoned out into a 1 liter stainless steel beaker using a peristaltic pump. A second portion of Shellsol T was added to the aqeuous magnetite slurry and treated the same was as the first portion of Shellsol T. The combined organic phases were heated in the stainless steel beaker to 130°C to get rid of any trace of water and then allowed to cool over a strong magnet. The cold liquid was subsequently filtered through a paperfilter (Munktell no. 3) while keeping the magnet in place on the bottom of the beaker while pouring the liquid into the filter funnel. To get most of the liquid out of the beaker some Shellsol T was added to the residue and allowed to mix without any stirring and then filtered as above. The resultant product is the superparamagnetic liquid in a low molecular weight hydrocarbon. Its content of magnetite is given by its saturation magnetization value.

The saturation magnetization value of the stable superparamagnetic liquid was determined by the following procedure.

A sample of superparamagnetic liquid was taken up in a capillary glass tube (6.6 ul Minicaps #900.11.66, sold by TG-Gruppen) by capillary force to a height of at least 15 mm, typically 25 mm, and the end of this capillary tube was subsequently sealed by dipping it into a melt of polyethylene or similar polymer or wax. This sample was then put in a magnetic susceptibility balance (produced by Johnson Matthey AB). The instrument reading was noted and recalculated by multiplying with a constant to give the saturation magnetization value. This constant was calculated by, using the procedure above, measuring several superparamagnetic liquids whose saturation magnetization values were accurately known from vibrating magnetometer measurements.

The following Examples further illustrate a process for making compounds of the present invention and how to use them as dispersants in metallic colloids.

Compounds of the present invention have been prepared in accordance with the present specification and particularly Example 1 below. Structures of compounds formed in accordance with the present invention are described in Table 1. The compounds listed in Table 1 were prepared by the method described in Example 1. Table 2 summarizes tests showing the utility of various compounds as dispersants in dioctyl phthalate carrier liquid as established by tests described in Example 4. Data showing the utility of the A-X-B dispersants in

"Priolube 3970" (produced by Unichema BV) tested in accordance with Example 5 is summarized in Table 3.

Example 1

PREPARATION OF A-X-B COMPOUNDS

In a 500 ml Erlenmeyer flask was placed 0.2 mol of the A group precursor (52.8 g of "DeSonic 6T" (tridecanol reacted with 6 moles of ethylene oxide, supplied by DeSoto Inc.)) and 0.2 mol of the X-B group precursor (20 g of succinic anhydride) along with 200 ml of xylene and 5 drops of pyridine. The mixture was agitated gently while it was heated to 150° C on a hot plate and the clear solution was held at this temperature for 2 hours. The solution was allowed to cool at room temperature before it was diluted with additional xylene to a final volume of 500 ml. The solution was then 0.4 molar of an A-X-B dispersant in xylene. This dispersant is identified as dispersant number 2 in Table 1.

The procedure described in Example 1 was used for the preparation of the A-X-B dispersants whose composition are described in Table 1.

Table 1

Dispersants with the general structure: $RO(CH_2CHYO)_n(CO)(CH_2)_pCOOH$

When succinic anhydride is the X-B group precursor, R will be an alkyl group, p will be 2 while glutaric anhydride will give p = 3. Nos. 23 and 24 have Y = hydrogen and methyl while the others have Y = hydrogen.

| Dispersant No. | Trade name of A group precursor | Supplier | No. of C atoms in R | Number of EO units, n | X-B group precursor |
|---|---|---|---|---|---|
| 1 | DeSonic DA-4 | DeSoto Inc. | 10 | 4 | Succinic anhydride |
| 2 | DeSonic 6T | DeSoto Inc. | 13 | 6 | Succinic anhydride |
| 3 | DeSonic DA-6 | DeSoto Inc. | 10 | 6 | Succinic anhydride |
| 4 | Alfonic 1012-60 | Vista Chem. Co. | 10-12 | 5.7 | Succinic anhydride |
| 5 | Alfonic 1412-60 | Vista Chem. Co. | 12-14 | 7 | Succinic anhydride |
| 6 | Trycol 5967 | Emery Industries | 12 | 12 | Succinic anhydride |
| 7 | Trycol 5963 | Emery Industries | 12 | 8 | Succinic anhydride |
| 8 | Trycol 5941 | Emery Industries | 13 | 9 | Succinic anhydride |
| 9 | Trycol 5943 | Emery Industries | 13 | 12 | Succinic anhydride |
| 10 | Neodol 23-6.5 | Shell Chem. Co. | 12-13 | 6-7 | Succinic anhydride |
| 11 | Neodol 25-12 | Shell Chem. Co. | 12-15 | 12 | Succinic anhydride |
| 12 | Neodol 91-8 | Shell Chem. Co. | 9-11 | 8 | Succinic anhydride |
| 13 | Butyl carbitol | Aldrich Chem. Co. | 4 | 2 | Succinic anhydride |
| 14 | Butyl cellosolve | Aldrich Chem. Co. | 4 | 1 | Succinic anhydride |
| 15 | Ethyl carbitol | Aldrich Chem. Co. | 2 | 2 | Succinic anhydride |
| 16 | Ethyl cellosolve | Aldrich Chem. Co. | 2 | 1 | Succinic anhydride |
| 17 | Brij 30 | ICI Specialty Chem. | 12 | 4 | Succinic anhydride |
| 18 | Brij 52 | ICI Specialty Chem. | 16 | 2 | Succinic anhydride |
| 19 | Brij 92 | ICI Specialty Chem. | 18 | 2 | Succinic anhydride |
| 20 | Genapol X-030 | Hoechst AG | iso 13 | 3 | Succinic anhydride |
| 21 | Genapol X-050 | Hoechst AG | iso 13 | 5 | Succinic anhydride |
| 22 | Genapol X-060 | Hoechst AG | iso 13 | 6 | Succinic anhydride |
| 23 | Tergitol MinFoam 1X | Union Carbide Corp. | 12-14 | Note 1 | Succinic anhydride |
| 24 | Tergitol MinFoam 2X | Union Carbide Corp. | 13-14 | Note 1 | Succinic anhydride |
| 25 | Neodol 25-12 | Shell Chem. Co. | 12-15 | 12 | Glutaric anhydride |
| 26 | DeSonic DA-4 | DeSoto Inc. | 10 | 4 | Glutaric anhydride |
| 27 | Berol 07 | Berol Kemi AB | 16-18 | 18 | Succinic anhydride |
| 28 | Tergitol TMN-3 | Union Carbide Corp. | 12 Note 2 | 3 | Succinic anhydride |

Note 1. These are mixture of ethylene oxide and propylene oxide.
Note 2. TMN stands for trimethylnonyl.

## Example 2

## TITRATION OF A-X-B DISPERSANTS

Exactly 4.00 ml of the 0.4 molar xylene solution of the A-X-B dispersant number 2, Table 1, prepared in Example 1, was placed in a 50 ml beaker along with 10 ml of ethanol and 10 ml of water. The mixture was stirred vigorously and titrated with 0.1 molar sodium hydroxide, recording the pH of the mixture after each addition of sodium hydroxide. The titration curve is shown in Figure 1.

## Example 3

## TITRATION OF DEXTROL OC-70, AN ACID PHOSPHORIC ACID ESTER

Exactly 2.00 ml of a Dextrol OC-70 solution in xylene (200 g Dextrol OC-70 in 500 ml of xylene) was placed in a 50 ml beaker along with 10 ml of ethanol and 10 ml of water. The mixture was stirred vigorously and titrated with 0.1 molar sodium hydroxide, recording the pH of the mixture after each addition of sodium hydroxide. The titration curves a Dextrol OC-70 acid phosphoric acid ester dispersant and dispersant number 2 of Table 1 are shown in Figure 1. The calculated $pk_a$ values are 2.6 for the acid phosphoric acid ester dispersant and 6.7 for dispersant number 2 of Table 1.

## Example 4

## EVALUATION OF A-X-B DISPERSANTS IN DIOCTYL PHTHALATE CARRIER LIQUID

The following general procedure was utilized to evaluate certain A-X-B dispersants and the materials utilized as well as the results are summarized in Table 2.

A total of 23 g of oleic acid coated magnetite was allowed to peptize into approximately 200 ml of xylene and 80 ml of the 0.4 molar A-X-B dispersant solution prepared according to the procedure of Example 1 was added with stirring. The mixture was heated to about 110°C in a stream of air to evaporate the xylene. The residue was cooled to about 30°C and washed with a minimum of three consecutive 200 ml portion of acetone, each time collecting the magnetite particles on the bottom of the beaker over a magnet. Acetone washing was continued until the acetone extracts were clear and colorless. This process served to remove any excess A-X-B dispersant as well as any particles coated by the dispersant which may be dispersable in acetone.

A quantity of about 100 ml of ethyl acetate was added to the washed particles and they were heated to evaporate acetone. A volume of 50 ml of the carrier liquid was added to the ethyl acetate slurry and the mixture was heated to 110°C in a stream of air to evaporate the ethyl acetate. The resulting superparamagnetic liquid was placed in a beaker over a magnet in a 65°C oven for 24 hours, then filtered away from the particles too large to be stabilized by the dispersant and which were attracted to and held on the bottom of the beaker by the magnet.

Table 2

EVALUATION OF DISPERSANTS IN DIOCTYL PHTHALATE

| Dispersant No. (from Table 1) | Dispersed magnetite in acetone | Sat. magnetization value (gauss) of superpara-magnetic liquid |
|---|---|---|
| 16 | no | no colloid formed |
| 14 | no | no colloid formed |
| 15 | no | no colloid formed |
| 13 | no | 193 |
| 1 | no | 248 |
| 4 | no | 230 |
| 2 | no | 242 |
| 10 | no | 236 |
| 9 | yes | 180 (excess. disp.) |
| 6 | yes | gel (excess. disp.) |
| 11 | yes | gel (excess. disp.) |
| 5 | no | 243 |
| 7 | no | 263 |
| 3 | no | 214 |
| 8 | no | 212 |
| 12 | no | 230 |
| 27 | yes | gel |
| 25 | yes | gel |

Example 5

EVALUATION OF A-X-B DISPERSANTS IN "PRIOLUBE 3970" CARRIER LIQUID

This general procedure was utilized to evaluate certain A-X-B dispersants and the materials utilized as well as the results are summarized in Table 3.

A total of 100 ml of a 200 gauss superparamagnetic liquid consisting of oleic acid coated magnetite particles dispersed in Shellsol T prepared according to the procedure given in the Procedure For Preparing Superparamagnetic Liquid Consisting of Fatty Acid Coated Magnetite, was placed in a 400 ml beaker and about 100 ml of acetone was added to cause flocculation of the colloid. The magnetite particles were collected on the bottom of the beaker and kept there by placing a strong magnet under the beaker and they were washed with an additional volume of 200 ml of acetone. To the residue was added about 200 ml of xylene and 80 ml of the 0.4 molar A-X-B dispersant solution prepared according to the procedure of Example 1. The mixture was heated at about 110°C in a stream of air to evaporate the xylene. The residue was cooled to about 30°C and washed with a minimum of three consecutive 200 ml portions of acetone, each time collecting the magnetite particles on the bottom of the beaker over a magnet. Acetone washing was continued until the acetone extracts were clear and colorless. This process served to remove any excess A-X-B dispersant as well as any particles coated by the dispersant which may be dispersable in acetone.

A quantity of about 100 ml of ethyl acetate was added to the washed particles and they were heated to evaporate acetone. A volume of 50 ml of the carrier liquid was added to the ethyl acetate slurry and the mixture was heated to 100°C in a stream of air to evaporate the ethyl acetate. The resulting superparamagnetic liquid was placed in a beaker over a magnet in a 65° oven for 24 hours, then filtered away from the particles too large to be stabilized by the dispersant and which were attracted to and held on the bottom of the beaker by the

magnet.

Table 3

EVALUATION OF DISPERSANTS IN "PRIOLUBE 3970"

| Dispersant No. (from table 1) | Dispersed magnetite in acetone | Sat. magnetization value (gauss) of superpara-magnetic liquid |
|---|---|---|
| 1 | no | 280 |
| 4 | no | 305 |
| 5 | no | 301 |
| 2 | no | 316 |
| 10 | no | 316 |
| 20 | no | 176 |
| 21 | no | 335 |
| 22 | no | 335 |
| 17 | no | 250 |
| 18 | no | 40 |
| 19 | no | 167 |
| 28 | no | 126 |
| 23 | yes (note 1) | 315 |
| 24 | yes (note 1) | 319 |
| 12 | no | gel |
| Akypo RLM 45 | no | 319 |
| Akypo RLM 100 | no | 320 |

Note 1. In these preparations the coated particles were washed with methanol to remove the excess dispersant.

Table 4

VISCOSITY OF SUPERPARAMAGNETIC LIQUIDS USING "PRIOLUBE 3970" CARRIER

| Dispersant No. (from table 1) | Sat. magnetization value (gauss) of superpara-magnetic liquid | Viscosity at 25°C in centipoise (cP) |
|---|---|---|
| 2 | 316 | 82.4 |
| 10 | 316 | 79.5 |
| 21 | 335 | 103.6 |
| 22 | 335 | 84.4 |

Table 5

EVALUATION OF SOME CARRIER LIQUIDS USEFUL IN THE PRACTICE OF THE INVENTION

| Carrier liquid | Supplier | Fatty acid test | Useful under this invention |
|---|---|---|---|
| Dioctyl phthalate | Malmsten & Bergwall | negative | yes |
| Dibutyl sebacate | Ciba-Geigy | negative | yes |
| Priolube 3970 | Unichema Chemie | negative | yes |

Comparison of the data in Tables 1 and 2 show that the number of ethylene oxide units in the A group may have a significant effect not only on the ability of the dispersant to form a stable superparamagnetic liquid with the carrier but also on the physical properties of the superparamagnetic liquid itself.

For example, dispersant 14 which was formed from butoxyethanol (one ethylene oxide unit) did not form a superparamagnetic liquid in dioctyl phthalate, whereas dispersant 13 which was formed from butoxyethoxyethanol (two ethylene oxide units) did. In general, dispersants in Table 1 with A groups containing from about two to about nine ethylene oxide units formed stable colloidal suspensions in dioctyl phthalate, but did not in acetone. Dispersants 6, 9, 11, and 27 form colloidal suspensions in acetone but form a thermally reversible gel in dioctyl phthalate at room temperature.

Although applicants do not wish to be bound by any particular theory or explanation, it is believed that the very long A groups of these dispersants are not well solvated by the carrier liquid and therefore tend to associate with other long A groups on other particles. These attractions are weak and thermally reversible, but are sufficient to immobilize the coated magnetite at lower temperatures and allow the formation of the gel. It may also be possible that the presence of the excess dispersant promoted the formation of the gel since it was not possible to remove excess dispersant by acetone washing as described by the procedure of Example 5.

The sensitivity of the interaction between the A group of the dispersant and the solvent is further illustrated by the data in Table 3 in which "Priolube 3970" (Unichema Chemie B.V.), a trimethylolpropane triester is used as the carrier for the superparamagnetic liquid.

Comparing the data in Table 3 with the data of Table 2 indicates that the solubility characteristics of "Priolube 3970" are substantially different from those of dioctyl phthalate. For example dispersant 12 with eight ethylene oxide units in the A group formed a gel in "Priolube 3970", while dispersant 7 with eight ethylene oxide units in the A group formed a stable superparamagnetic liquid in dioctyl phthalate.

The saturation magnetization values of table 3 show that dispersant 2, 10, 21, or 22 would be useful as dispersants in "Priolube 3970". However, a choice of the most useful material should also include consideration of the viscosity of the superparamagnetic liquid as shown in Table 4.

These data show that either dispersant 10 or dispersant 22 would be a preferred dispersant for many applications, particularly use in dioctyl phthalate. However, dispersant 10 contains an average of 6-7 ethylene oxide units, dangerously close to the average of eight ethylene oxide units of dispersant 12 which formed a gel. Therefore, dispersant 22 which has 6 ethylene oxide units is the most preferred material.

The selection of a dispersant for a particular carrier liquid requires consideration of a number of factors described and explained in the foregoing specification. The ensuing paragraphs provide additional information useful in designing a dispersant for a particular carrier liquid.

A suitable dispersant is one that produces an ideal stable colloid (the particles undergo elastic collisions) and that produces low colloid viscosity at any specific magnetization value.

It is quite difficult to predict the performance properties of a particular dispersant in a particular carrier liquid. For example, although oleic acid will produce a colloidal suspension of magnetite in a light weight liquid hydrocarbon, suoh as xylene, it will fail to produoe a colloidal suspension of magnetite in heavier liquid hydrocarbons such as 6 cst poly(alpha olefin) oil. In order, therefore, to select a dispersant which forms the best superparamagnetic liquid in a specific carrier liquid, considering stability of the colloidal suspension and viscosity of the colloid at any given value of saturation magnetization, i.e., the volume content of magnetic material, it is ordinarily necessary to test a variety of dispersants with similar but somewhat different structure.

With a subdomain size particle of magnetite, the length of the oil soluble portion of a dispersant acid, when dissolved in the carrier liquid, must be at least about 0.2 times the diameter of the magnetic particle in order to maintain the magnetic particle in stable suspension. If the length of the oil scluble portion of the dispersant when dissolved in the carrier is less than about 0.2 times the diameter of the magnetic particle, the particles can approach closely enough so that the attractive force between the particles will overcome the repulsive force produced by the dispersant and the particles will agglomerate.

The saturation magnetization value of the supermagnetic liquid is determined by the volume content of magnetic material in the superparamagnetic liquid. The viscosity of the superparamagnetic liquid is, if it is one which is or approaches being an ideal colloid, a function of carrier liquid viscosity and the total disperse phase

volume. The disperse phase volume is that of the magnetic material plus the phase volume taken up by the A groups stretched out form the surface of the magnetic material. Therefore, when the A groups are longer than required to provide stability to the dispersed magnetic particles, the total disperse phase volume and therefore the colloid viscosity will be greater than it needs to be.

When compounds of the present invention are to be used as additives in rust inhibiting oils or lubricant additives, it is important to ensure that the oil and the additive can be washed away from the metal surface so the metal surface can be inspected or treated. In order to determine whether the oil/compound mixture can be washed off the metal surface by aqueous ammonia, the so- called "water break test" can be performed. After washing with aqueous ammonia, the metal surface is dipped in water to see if the water forms small droplets or a continuous film on the metal surface. In the latter case the oil compound mixture has been washed off the surface.

The removal of the oil containing additives of the present invention from a surface will be aided if the additive is a good emulsifying agent.

To show that the compound is a good emulsifier, the following test has proven useful:

1. Mix the oil and alkaline water (25% oil/75% water, made alkaline by ammonium hydroxide) in a test tube and shake. Note the time required before two distinct phases are formed.

2. Mix the oil and alkaline water as above in a test tube with a bit of the compound selected, and shake. Note the time required before the emulsion breaks. If the emulsion breaks only very slowly, the compound will be a good emulsifier. Obviously, the best emulsifiers will be those that form an emulsion and form an emulsion that does not break.

It will be apparent to those skilled in the art that various modifications and variations can be made in the products and processes of the present invention without departing from the scope of spirit of the invention. Thus, it is intended that the present invention cover modifications and variations thereof provided they come within the scope of the appended claims and their equivalents.

## Claims

1. A chemical compound of the formula:

A-X-B

wherein A is derived from a non-ionic surface active agent and is soluble in high molecular weight non-polar oil or a polar lubricant when said compound is bonded to metal, B is an organic carboxylic group and X is a connecting group linking A to B wherein X comprises at least two carbon atoms.

2. A chemical compound according to claim 1, wherein A is derived from non-ionic surface active agents selected from the group consisting of ethoxylated alcohols, ethoxylated alkyl phenols, ethoxylated fatty acids, ethoxylated amides, ethoxylated amines and ethylene oxide/propylene oxide polymers.

3. A chemical compound according to claim 2, wherein:

A is $RO(CH_2CHYO)_n$ , in which R is a linear or branched alkyl or alkylene chain with 2-25 carbons or an alkylated aromatic group;

n is at least 1 to 19; and

Y is hydrogen or methyl.

4. A chemical compound according to claim 2 wherein A is:

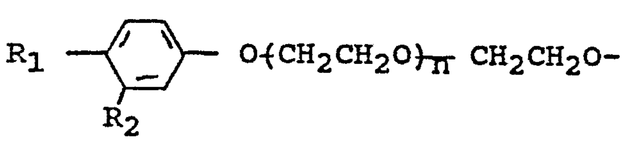

wherein, $R_1$ = tertiary $C_8$, or $C_9$;

$R_2$ = H or $C_8$ or $C_9$; and

n = 1 to 19.

5. A chemical compound according to claim 2 wherein A is:

$R-\overset{O}{\overset{\|}{C}}-O(CH_2CH_2O)_n\ CH_2CH_2O-$;

n = 1 to 19;

R = $C_{11}$ to about $C_{17}$ carboxylic acid, preferably lauric, myristic, palmitic, oleic, stearic, or isostearic acid.

6. A compound as defined in claim 2 wherein A is:

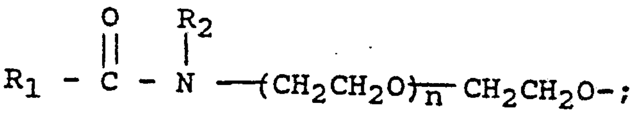

$R_1$ is a fatty acid such as lauric, myristic, palmitic, oleic, stearic, or isostearic acid;
n = 0 to 29;
$R_2 = CH_3$ or $-(CH_2CH_2O)_n-$ $CH_2CH_2OH$ and is preferably $CH_3$.

7. A compound as defined in claim 2 wherein A is:

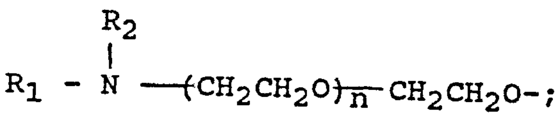

$R_1$ can be an alkyl group with from about 4 to about 25 carbon atoms;
$R_2$ can $= R_1$ or $R_2$ can be $-CH_3$ or $-(CH_2CH_2O)_n-$ $-CH_2CH_2OH$;
n = 1 to 29.

8. A compound as defined in claim 2 wherein A is:

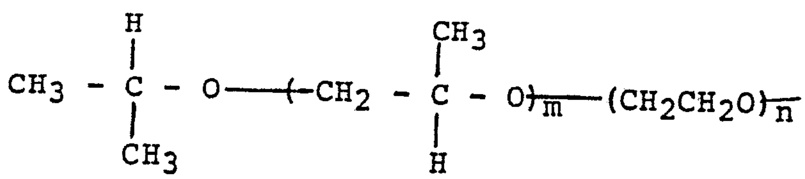

wherein m and n are greater than 1.

9. A chemical compound according to claims 3, 4, 5, 6, 7 or 8, wherein X is:

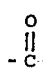 $-(CH_2)_p-$ where p = 1-8.

10. A chemical compound according to claim 9, wherein p is 2 or 3.

11. Chemical compounds according to claims 3, 4, 5, 6, 7 or 8 wherein X is $-(CH_2)_q-$ where q = 2-8.

12. A chemical compound according to claims 3, 4, 5, 6, 7 or 8, wherein X is an aromatic or substituted aromatic group according to the formula:

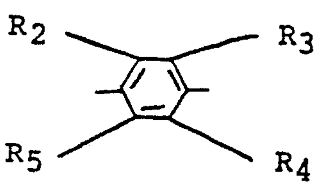

wherein $R_2$, $R_3$, $R_4$ and $R_5$ are the same or different and are hydrogen, alkyl groups with 1-25 carbons, halogen or additional $R-O(CH_2CHYO)_n$ groups wherein Y is hydrogen or methyl and is 1-19.

13. A chemical compound according to claims 3, 4, 5, 6, 7 or 8, wherein X is a perfluorinated chain having 2-12 carbon atoms.

14. A chemical compound according to claim 3, wherein R is an alkyl group with 4-15 carbons, Y is hydrogen and n = 2-10 and wherein X is

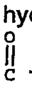 $-(CH_2)_p$ wherein p is 2 or 3.

15. Chemical compositions comprising lubricating oil or corrosion inhibiting oil and an additive comprising the compounds of claim 3 which is dissolved by said oil.

16. Chemical compositions comprising lubricating oil or corrosion inhibiting oil and an additive

comprising compounds of claim 9.

17. Chemical compositions comprising lubricating oil or corrosion inhibiting oil and an additive comprising compounds of claim 10.

18. Chemical compositions comprising lubricating oil or corrosion inhibiting oil and an additive comprising compounds of claim 12.

19. Chemical compositions comprising lubricating oil or corrosion inhibiting and $R_1O(CH_2CH_2O)_n CH_2 \overset{\overset{O}{\|}}{C} OH$ as an additive for said oil.

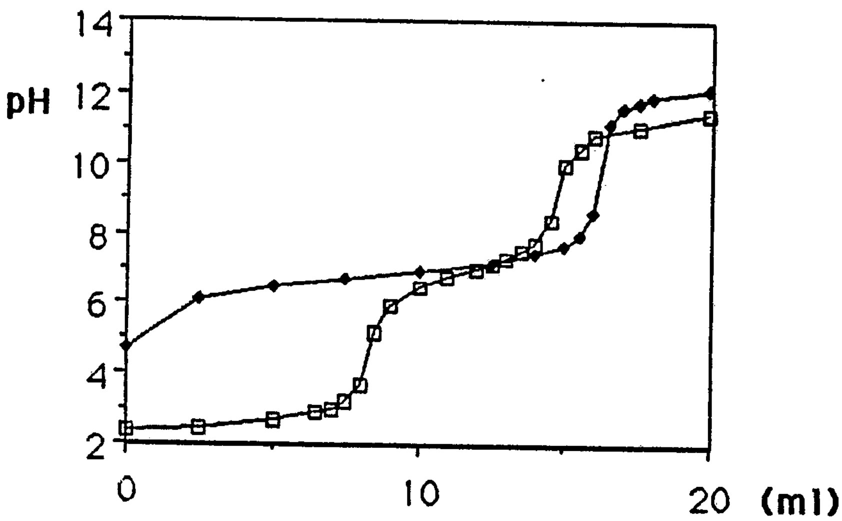


Figure 1

Figure 1. Titration curves of Dextrol OC-70 ( -⊟- ) acid phosphoric acid ester dispersant and dispersant number 2 ( -◆- ) of Table 1. The horizontal axis shows the volume of added 0.1 molar sodium hyroxide solution, while the vertical axis shows the pH.
pKa values: Dextrol OC-70 = 2.6, dispersant number 2 = 6.7.